## Europäisches Patentamt
## European Patent Office
## Office européen des brevets

(19)

(11) Numéro de publication: **0 244 295**

**A1**

(12)

# DEMANDE DE BREVET EUROPEEN

(21) Numéro de dépôt: **87400870.9**

(22) Date de dépôt: **16.04.87**

(51) Int. Cl.³: **C 07 K 15/14**
C 07 H 3/06, A 61 K 39/395
A 61 K 39/00, G 01 N 33/569
A 61 K 39/44, C 12 P 21/00
C 12 N 15/00

(30) Priorité: **30.04.86 FR 8606281**

(43) Date de publication de la demande:
**04.11.87 Bulletin 87/45**

(84) Etats contractants désignés:
**AT BE CH DE ES FR GB GR IT LI LU NL SE**

(71) Demandeur: **INSTITUT PASTEUR**
**28, rue du Docteur Roux**
**F-75724 Paris Cédex 15(FR)**

(71) Demandeur: **INSTITUT PASTEUR DE LILLE**
**Rue du Professeur A. Calmette BP 245**
**F-59019 Lille Cédex(FR)**

(71) Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Grzych, Jean-Marie**
**Les Tilleuls 1 rue Meunier, Apt. 202**
**F-59700 Marcq(FR)**

(72) Inventeur: **Capron, André**
**58 rue du Capitaine Jasmin**
**F-59133 Phalempin(FR)**

(72) Inventeur: **Dissous, Colette**
**7 Allée des Symphorines**
**F-59262 Sainghin en Mélantois(FR)**

(74) Mandataire: **Ores, Irène et al,**
**CABINET ORES 6, Avenue de Messine**
**F-75008 Paris(FR)**

(54) Immonugène isolé de mollusques, son procédé de préparation, réactifs de diagnostic des bilharzioses humaines, vaccins contre les schistosomiases et compositions immunisantes comprenant ledit immunogène.

(57) Glycoprotéine extraite de mollusques, notamment de Megathura crenulata, savoir l'hémocyanine, qui présente les mêmes propriétés antigéniques que l'antigène 38 KD isolé de la surface de schistosomule de S. mansoni; son épitope oligosaccharidique.

Sérum protecteur à l'égard des infections par S. mansoni, provenant d'un mammifère immunisé par l'hémocyanine de mollusque précitée ou son épitope oligosaccharidique.

Composition immunogène contenant ladite glycoprotéine ou son épitope oligosaccharidique et ses applications en tant que réactif de diagnostic, vaccin contre les bilharzioses, agent immunisant, etc ...

EP 0 244 295 A1

Croydon Printing Company Ltd

- 1 -

La présente invention est relative à un immunogène isolé de divers mollusques et notamment d'un mollusque marin dénommé Megathura crenulata et à son procédé de préparation, ainsi qu'à son utilisation en tant que réactif de diagnostic des bilharzioses humaines, vaccin contre les schistosomiases et agent immunisant.

Des travaux antérieurs des Inventeurs ont montré qu'un antigène de poids moléculaire 38 KD présent à la surface du schistosomule, est une glycoprotéine qui constitue l'un des immunogènes majeurs des différents systèmes d'infections expérimentales [DISSOUS et CAPRON, 1982 "Protides of biological Fluids"; Peeters H. Ed., Elsevier, Amsterdam - New-York, p. 179-182] et qu'elle suscite chez 97% de sujets brésiliens infectés par S. mansoni la production d'anticorps spécifiques [DISSOUS et Al., 1984, J. INFECT. DIS, 149, p. 227-233]. Cet antigène 38 KD est, d'autre part, reconnu par un anticorps monoclonal de rat protecteur IPL Sm1 d'isotype IgG2a [DISSOUS et Al. J. IMMUNOL., 1982, 129, p.2232-2234] doué d'une haute activité cytotoxique vis-à-vis du schistosomule en présence d'éosinophiles, reproduisant ainsi le mécanisme d'ADCC décrit précédemment par CAPRON et Al. [EUR. J. IMMUNOL., 1978, 8, p. 127-133] comme l'un des mécanismes essentiels de l'immunité à la schistosomiase expérimentale du rat. Cet anticorps s'est d'autre part révélé doté d'une intense activité protectrice par transfert passif, établissant chez le rat un niveau de protection comparable à celui conféré par l'anticorps polyclonal ou par une première infection [GRZYCH et Al. J. IMMUNOL., 1982, 129, p. 2739-2743].

Comme les schistosomes ne se multiplient pas in vitro, le matériel parasitaire disponible est insuffisant pour réaliser des préparations utilisables en tant qu'immunogène.

Cependant si l'on considère le nombre élevé de malades atteints de schistosomiase - ou bilharziose - (au moins 200 millions), il faudrait pouvoir disposer de sources antigéniques dont l'importance quantitative soit à la mesure de l'immunoprophylaxie des infections par S. mansoni qu'il faudrait pouvoir mettre en oeuvre.

Des travaux effectués par l'un des Inventeurs il y a environ 20 ans [ANNALES DE L'INSTITUT PASTEUR, A. CAPRON et Al. "Les Antigènes de Schistosoma mansoni", Novembre 1965, Tome 109, p. 798-810] ont démontré l'existence de parentés antigéniques entre Schistosoma mansoni et son hôte intermédiaire, le mollusque Biomphalaria glabrata. La découverte de ce phénomène de mimicrie moléculaire a été à l'origine de la démonstration du fait que l'existence de déterminants antigéniques communs au parasite et à son hôte intermédiaire est un phénomène général et d'une signification biologique essentielle dans la compréhension des relations hôte-parasite [CAPRON et Al. PATH. BIOL. 1968, Vol. 16, N°3-4, p. 121-138] : il s'agit d'une immunoadaptation du parasite fondée sur l'induction de systèmes protéiniques antigéniquement identiques à ceux de l'hôte et dont la mise en jeu aboutit à l'atténuation des disparités hôte-parasite. Ces travaux ont été confirmés récemment par DISSOUS et Al. qui ont démontré que l'épitope glycanique de la molécule protectrice 38 KD du schistosome est exprimé chez son hôte intermédiaire Biomphalaria glabrata.

Ces travaux ont suggéré aux Inventeurs que des mollusques, hôtes intermédiaires de parasites, pourraient constituer des sources abondantes de matériel antigénique propre à déterminer une immunité protectrice à l'égard d'affections parasitaires et que ces sources pourraient être constituées de façon spécifique par des épitopes glycaniques isolés de ces hôtes intermédiaires.

Il a été montré que l'antigène de poids moléculaire 38 KD présent à la surface du schistosomule est une glycoprotéine qui représente l'un des immunogènes majeurs des différents systèmes d'infections expérimentales (DISSOUS et CAPRON : document cité).

La présente invention est basée sur le fait que cet

- 3 -

immunogène correspond à une glycoprotéine de mollusque et notamment d'un mollusque marin, Megathura crenulata, très ancien puisque l'on trouve la trace de son existence et de son évolution dans des gisements géologiques vieux de centaines de millions d'années et que cette glycoprotéine présente une activité immunisante protectrice.

La présente invention a pour objet une glycoprotéine extraite de mollusques, savoir l'hémocyanine, qui est caractérisée en ce qu'elle présente les mêmes propriétés antigéniques que l'antigène 38 KD isolé de la surface du schistosomule de S. mansoni.

Des observations dont il sera rendu compte plus loin ont en effet permis de mettre en évidence l'existence d'une communauté antigénique entre certaines structures présentes à la surface du schistosomule et certains déterminants constitutifs de la molécule d'hémocyanine. La communauté antigénique entre la glycoprotéine 38 KD et l'hémocyanine de mollusques tels que Megathura crenulata notamment, a, d'autre part, été confirmée par l'analyse de fractions purifiées d'hémocyanine en gel de polyacrylamide, suivie d'un "Western blotting" démontrant la reconnaissance de structures moléculaires d'environ 150 à 200 KD par un anticorps monoclonal, dénommé IPL Sm1 et le sérum d'animaux infectés par Schistosoma mansoni.

L'anticorps monoclonal IPL Sm1 est un anticorps monoclonal de rat, d'isotype IgG2a, obtenu par hybridation cellulaire entre des cellules spléniques de rats LOU immunisés contre Schistosoma mansoni et des cellules myélomateuses de rat IR983, en appliquant la technique décrite par GRZYCH et Al. [J. IMMUNOL., 1982, 129, p. 2739-2743].

Les expériences de déglycosylation pratiquées d'une part sur l'antigène 38 KD spécifique de l'anticorps IPL Sm1 et d'autre part sur l'hémocyanine de mollusques, dans le cadre de la caractérisation biochimique de l'épitope commun à l'hémocyanine et à l'antigène 38 KD, ont montré, comme on le verra plus loin, la nature oligosaccharidique de l'épitope porté par l'antigène 38 KD du schistosomule et par l'hémocyanine de mollusque et reconnu par l'anticorps monoclonal protecteur IPL Sm1.

La présente invention a donc également pour objet un oligosaccharide caractérisé en ce qu'il est commun à l'antigène 38 KD du schistosomule et à l'hémocyanine de mollusque, et en ce qu'il est reconnu par l'anticorps monoclonal protecteur IPL Sm1 comme étant l'épitope responsable du pouvoir antigénique de l'antigène 38 KD de schistosomule et de l'hémocyanine de mollusque.

La présente invention a en outre pour objet un sérum protecteur à l'égard des infections par Schistosoma mansoni, caractérisé en ce qu'il est constitué par un sérum de mammifère immunisé par de l'hémocyanine de mollusque ou par son épitope oligosaccharidique, en ce qué ce sérum contient des anticorps capables d'induire en présence d'éosinophiles de mammifères du même genre que ceux dont provient le sérum, des niveaux significatifs de cytotoxicité, de l'ordre de 46 à 94% environ, comparables à ceux obtenus dans le cas des sérums d'infection par S. mansoni ou par l'anticorps monoclonal protecteur IPL Sm1, lequel sérum est apte à exercer une activité protectrice immunoprophylactique, à l'égard des infections par S. mansoni.

Selon un mode de réalisation avantageux du sérum conforme à la présente invention, ce sérum est un sérum de mammifère immunisé par l'hémocyanine de mollusques pris dans le groupe qui comprend notamment Megathura crenulata.

La présente invention a également pour objet une composition immunogène qui est caractérisée en ce qu'elle contient une quantité suffisante de la glycoprotéine définie ci-dessus ou de son épitope également défini plus haut, éventuellement associé(e) à un adjuvant convenable.

La présente invention a de plus pour objet un réactif de diagnostic simple, standardisable et de faible prix de

- 5 -

revient, apte à être utilisé pour la séroépidémiologie des bilharzioses humaines, caractérisé en ce que son constituant essentiel est l'immunogène ci-dessus, à savoir l'hémocyanine de mollusque, et notamment de mollusques pris dans le groupe comprenant Megathura crenulata, ou son épitope oligosaccharidique.

La présente invention a encore pour objet un vaccin contre les bilharzioses et notamment les bilharzioses humaines, caractérisé en ce qu'il contient une quantité vaccinante active d'hémocyanine de mollusque, et notamment de mollusques pris dans le groupe comprenant Megathura crenulata, ou de l'épitope oligosaccharidique de cette hémocyanine.

La présente invention a en outre pour objet un agent immunisant constitué par un complexe formé par le couplage d'anticorps anti-idiotypes spécifiques d'anticorps monoclonaux de mammifères anti-Schistosoma mansoni, à l'hémocyanine de mollusques, et notamment à l'hémocyanine de Megathura crenulata.

Le complexe ainsi formé entre l'hémocyanine de mollusque et les anticorps susdits a pour effet d'optimiser la réponse immune desdits anticorps vis-à-vis du schistosome.

Il était connu antérieurement à la présente invention d'utiliser l'hémocyanine du mollusque marin Megathura crenulata (KLH) comme protéine porteuse pour le couplage d'anticorps. La mise en évidence, dans le cadre de la présente invention, des propriétés antigéniques de l'hémocyanine vis-à-vis du schistosome de S. mansoni, augmente encore l'intérêt de l'utilisation de l'hémocyanine comme protéine porteuse d'anticorps, pour la réalisation d'immunisations vis-à-vis de peptides synthétiques.

La présente invention a en outre pour objet un anticorps monoclonal qui est caractérisé en ce qu'il est apte à se lier spécifiquement à des structures de nature glycanique de l'hémocyanine de Megathura crenulata, de masse moléculaire 150-200 KD.

0244295

- 6 -

Conformément à l'invention, l'anticorps monoclonal susdit, dénommé IPL Sm1, est obtenu à partir d'un hybridome déposé le 25 Avril 1986 sous le N° I-547 auprès de la Collection Nationale de Culture de Microorganismes (CNCM) tenue par l'INSTITUT PASTEUR.

La présente invention a également pour objet un hybridome utile pour la préparation des anticorps monoclonaux susdits, qui est caractérisé en ce qu'il est obtenu par fusion de cellules de rate de rats immunisés contre S. mansoni et de cellules myélomateuses de rat.

Un hybridome entrant dans le cadre de la présente invention a été déposé le 25 Avril 1986 sous le N° I-547 auprès de la CNCM.

La présente invention a, de plus, pour objet un antigène qui est caractérisé en ce qu'il est reconnu par l'anticorps monoclonal produit par un hybridome tel que défini plus haut.

La présente invention a également pour objet un coffret, trousse ou analogue de diagnostic prêt à l'emploi qui est caractérisé en ce qu'il contient, outre les tampons, supports et autres réactifs usuels permettant la réalisation d'un diagnostic par les méthodes usuelles telles qu'immunofluorescence, EIA, RIA et analogues : - une quantité suffisante de la glycoprotéine immunogène définie plus haut ou de son épitope oligosaccharidique; - une quantité suffisante d'un anticorps monoclonal tel que défini plus haut; - et un sérum de référence.

La communauté antigénique étroite entre l'antigène 38 KD du schistosome de Schistosoma mansoni et l'hémocyanine de mollusques a été mise en évidence par une série d'expériences qui sont décrites dans ce qui va suivre.

1 - Mise en évidence de la spécificité croisée

Dans une première série d'expériences, l'anticorps antiidiotype JM8-36 spécifique de l'anticorps monoclonal protecteur de rat IPL Sm1 [GRZYCH et Al., Nature, 1985, 316 : p. 74-76] a été couplé à une quantité égale d'hémocyanine de Megathura crenulata (KLH), par l'intermédaire du glutaraldéhyde. Deux séries de rats LOU/M mâles (180-200 g) ont ensuite été immunisées, l'une par le copolymère JM8-36/KLH (600 µg par rat), l'autre par 300 µg de KLH (grou-pes d'animaux témoins d'immunisation) par voie sous-cutanée dorsale. Une seconde injection a été réalisée dans les mêmes conditions quinze jours plus tard. Les animaux ont été saignés toutes les semaines après la première injection. La qualité de l'immunisation a été appréciée par la technique d'immunofluorescence indirecte sur coupes de schistosomules de S. mansoni à partir des sérums de rats immunisés.

Les résultats obtenus dans ces expériences ont démontré que si les sérums de rats immunisés par l'anticorps JM8-36 couplé à la KLH contiennent des anticorps spécifiques du schistosomule, une réaction positive est aussi observée dans le cas des sérums des rats ayant uniquement reçu de l'hémocyanine de M. crenulata.

2 - Caractérisation des spécificités antigéniques communes à la KLH et aux schistosomules

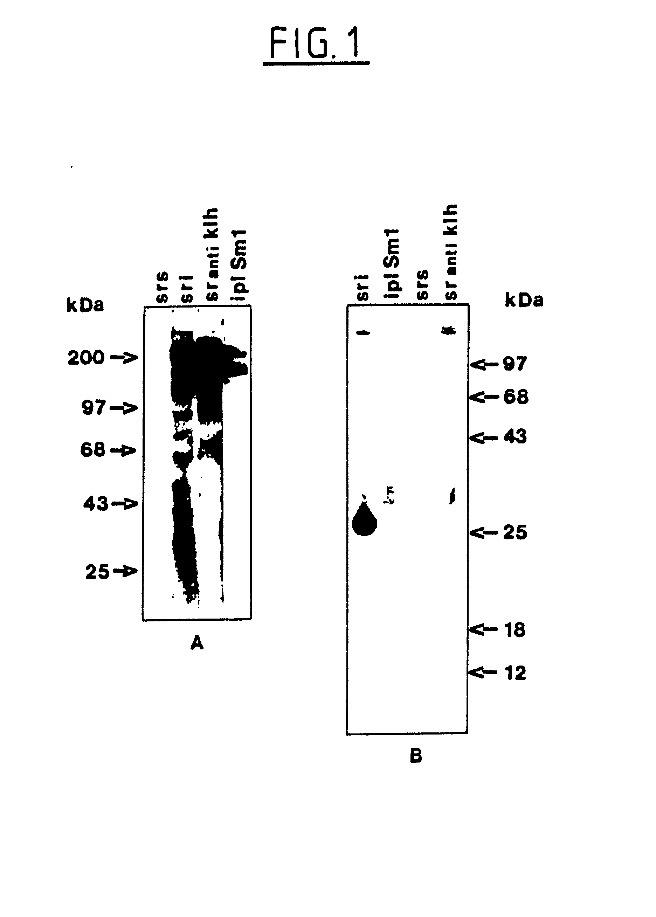

La spécificité anti-parasitaire des sérums de rats immunisés par la KLH a été envisagée grâce aux techniques d'immunoprécipitation. Ces réactions réalisées selon le protocole décrit par DISSOUS et Al. [Mol. Biochem. Parasitol., 1981, 3 : p. 215-225] ont révèlé que les rats immunisés par la KLH produisent des anticorps capables de se lier spécifiquement à un antigène de poids moléculaire 38 KD présent à la surface du schistosomule : cf. la Figure 1B qui représente l'immunoprécipitation des antigènes de surface des schisto-somules marqués par l'$^{125}$I par les différents anticorps de rat [DISSOUS et Al. MOL. BIOCHEM. PARASITOL. (1981), 3, p. 215-225]. Cet antigène 38 KD est d'autre part reconnu par un anticorps monoclonal de rat protecteur IPL Sm1 d'isotype IgG2a [DISSOUS et AL., J. Immunol., 1982, 129 : p.2232-2234] doué d'une haute activité cytotoxique vis-à-vis du schisto-

- 8 -

somule en présence d'éosinophiles, reproduisant ainsi le mécanisme d'ADCC décrit précédemment par [CAPRON et Al. Eur.
J. Immunol., 1978, 8 : p. 127-133] comme l'un des mécanismes
essentiels de l'immunité à la schistosomiase expérimentale du
rat.Cet anticorps s'est d'autre part révélé doté d'une intense activité protectrice par transfert passif établissant chez
le rat un niveau de protection comparable à celui conféré par
l'anticorps polyclonal ou par une première infection [GRZYCH
et Al. J. Immunol., 1982, 129 : p. 2739-2743].

La communauté antigénique existant entre la GP
38 KD et l'hémocyanine de M. crenulata a, d'autre part, été
confirmée par l'analyse de fraction purifiée de KLH en gel de
polyacrylamide, suivie d'un "Western blotting" démontrant la
reconnaissance de structures moléculaires d'environ 150 à
200 KD par l'anticorps monoclonal IPL Sm1 et le sérum d'animaux infectés par S. mansoni : cf. la Figure 1A qui représente l'analyse d'une fraction purifiée de KLH en gel de polyacrylamide-SDS 10% en conditions réductrices (ß-mercaptoéthanol), suivie d'un "Western blotting" en présence de 5 µl de
sérum de rat sain (srs), 5 µl de sérum de rat infecté par S.
mansoni depuis 100 jours (sri), 5 µl de sérum de rat immunisé
contre KLH (sr anti-KLH) ou 10 µg d'anticorps IPL Sm1, la révélation étant obtenue par des IgG de lapin anti-IgG de rat
marquées par l'$^{125}$I (5.10$^5$ cpm/piste).

3 - <u>Etude des activités biologiques des sérums de rats</u>
<u>immunisés par la KLH</u>

La relation étroite existant entre l'expression du
mécanisme de cytotoxicité dépendant d'éosinophiles et l'antigène 38 KD, a conduit à apprécier le potentiel cytotoxique
des sérums prélevés après immunisation par la KLH.

Les expériences de cytotoxicité réalisées dans les
conditions définies par CAPRON et Al. [Eur. J. Immunol.,
1978, 8 : p. 127-133] intéressant 12 sérums de rats immunisés
par la KLH, démontrent clairement l'existence d'anticorps capables d'induire en présence d'éosinophiles de rats des ni-

- 9 -

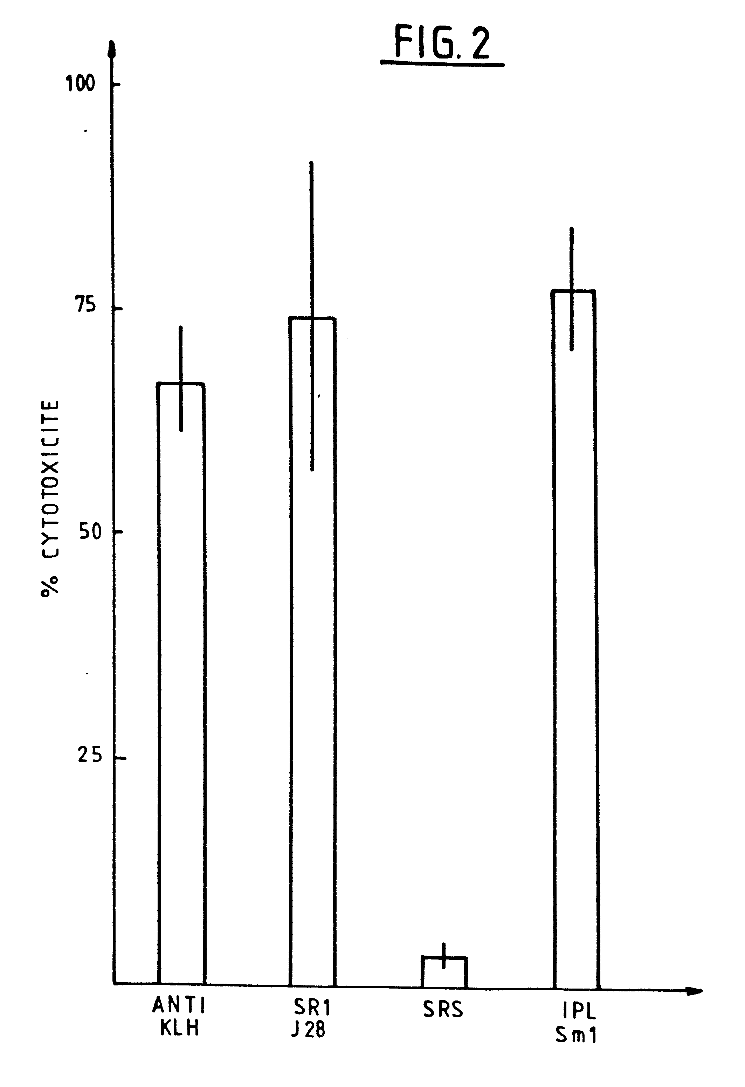

veaux significatifs de cytotoxicité (46 à 94%) comparables à ceux obtenus dans le cas des sérums d'infection ou par l'anticorps monoclonal protecteur IPL Sm1 : cf. la Figure 2 annexée qui montre la cytotoxicité dépendante d'éosinophiles.

Les expériences de cytotoxicité sont réalisées selon la technique de CAPRON et Al. (1978). 50 schistosomules de S. mansoni dans 50 µl de milieu MEM sont incubés en présence des différents sérums à tester, dilués au 1/4 dans le milieu MEM et chauffés 1 heure à 56°C; soit 100 µl de sérum de rat immunisé par la KLH (anti-KLH), soit 100 µl de sérum de rat infecté prélevé 28 jours après l'infection (sri J.28), soit 100 µl de sérum de rat sain (srs), soit 100 µl de sérum de rat portant la tumeur sous-cutanée de l'hybridome produisant l'anticorps monoclonal protecteur (IPL Sm1). Les schistosomules sont incubés 18 heures à 37°C en présence des différents sérums. Puis les cellules effectrices constituées de cellules péritonéales non adhérentes de rats sont ajoutées dans un rapport de 6000 cellules effectrices pour un schistosomule. Ces cellules effectrices contiennent 40 à 90% d'éosinophiles et 4 à 10% de mastocytes. Le pourcentage de mortalité est évalué après 24 et 48 heures d'incubation des schistosomules en présence des cellules effectrices, par observation au microscope.

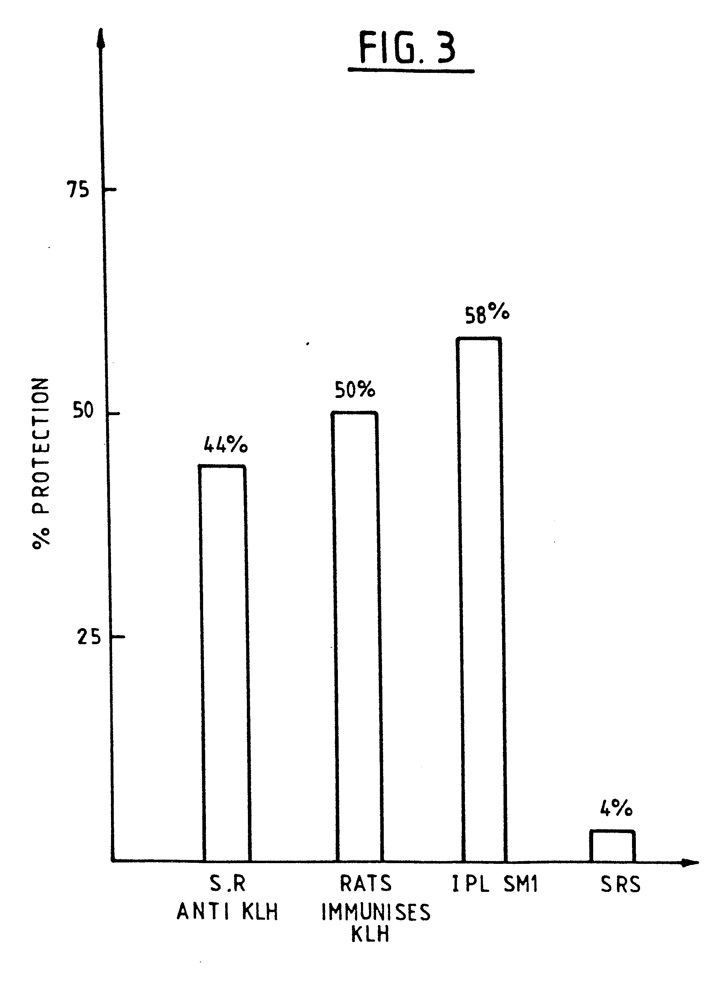

Les anticorps produits au cours de l'immunisation de rats par la KLH se sont, d'autre part, révélés dotés d'activité protectrice lorsqu'ils sont transférés passivement à l'animal, établissant ainsi des degrés de protection identiques à ceux précédemment décrits pour l'anticorps IPL Sm1. Fait plus important, les rats immunisés par la KLH se trouvent significativement protégés vis-à-vis de l'infection par S. mansoni, révélant ainsi les potentialités d'utilisation de la KLH dans la mise en oeuvre d'une immunoprophylaxie des infections par S. mansoni. La Figure 3 annexée représente l'activité protectrice des anticorps produits au cours de l'immunisation par la KLH, vis-à-vis de l'infection par Schistosoma mansoni.

Le pouvoir protecteur des anticorps produits au cours de l'immunisation par la KLH vis-à-vis de l'infection par Schistosoma mansoni est évalué 21 jours après l'infection par la technique de perfusion du foie [SMITHERS et Al., Parasitology, 1965, 55, p. 695] :

1) soit chez des rats LOU/M mâles ayant reçu par voie intraveineuse, 4 heures après l'infection par 800 cercaires de S. mansoni, 1,5 ml de sérum de rat immunisé par la KLH (sr anti-KLH), soit 1,5 ml de sérum de rat sain, soit 1,5 ml de liquide d'ascite de l'hybridome producteur de l'anticorps monoclonal protecteur IPL Sm1 (IPL Sm1).

2) soit chez des rats LOU/M mâles immunisés par la KLH et présentant au niveau sérique une réaction de cytotoxicité dépendante d'éosinophiles positive, puis infectés par 800 cercaires de S. mansoni.

3) Le pourcentage de protection est évalué par la formule suivante (A-B)/A x 100 où A = le nombre de vers obtenus chez les animaux traités par 1,5 ml d'une solution de NaCl à 0,9% et B le nombre de vers obtenus chez les rats traités par les différentes préparations sériques ou immunisés par la KLH.

4 - <u>Approche épitopique</u>

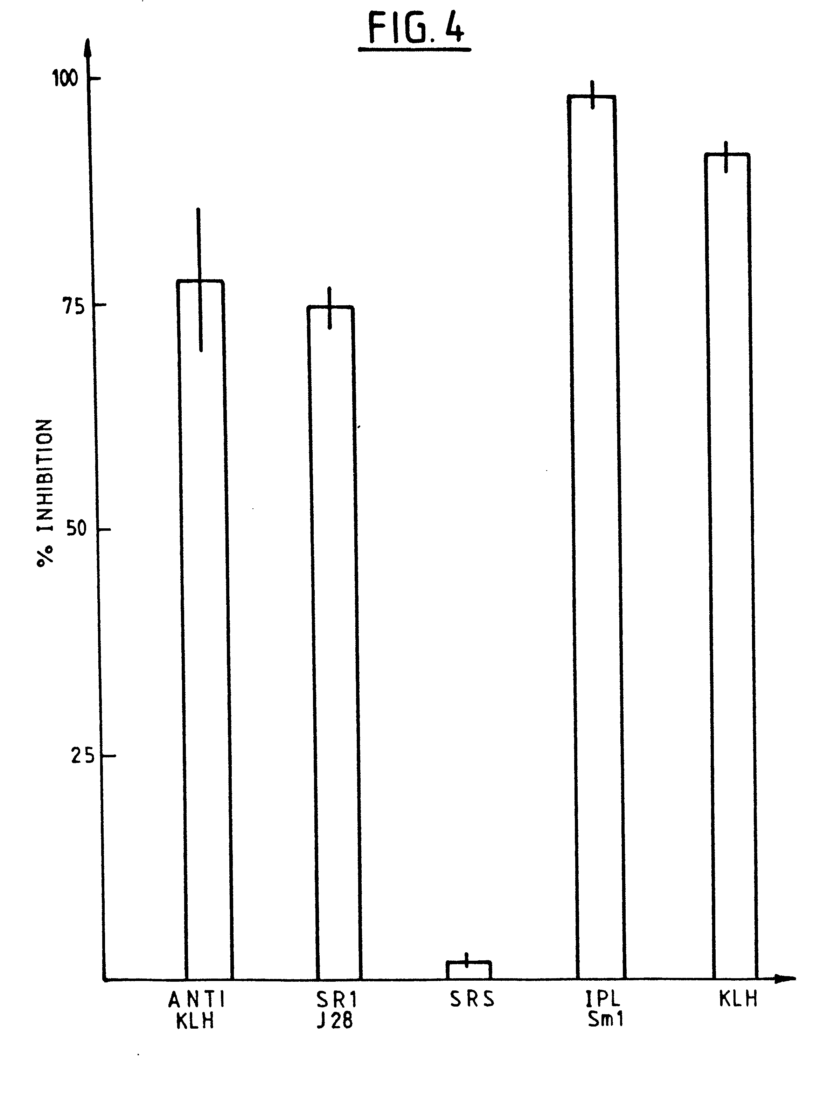

Disposant d'une sonde monoclonale spécifique de la molécule 38 KD (anticorps IPL Sm1), une approche fine du site de fixation des anticorps produits au cours de l'immunisation de rats par la KLH a été entreprise. Cette étude a été effectuée par la mise au point d'une technique fondée sur l'inhibition de la fixation de l'anticorps IPL Sm1 marqué à l'iode 125 sur son antigène cible 38 KD par les sérums d'animaux immunisés par la KLH. Les résultats présentés dans la Figure 4 démontrent clairement que les anticorps produits au cours d'une telle immunisation induisent des taux d'inhibitions identiques à ceux observés pour les sérums d'infection, l'anticorps IPL Sm1 "froid" ou la KLH purifiée.

La technique radioimmunologique d'inhibition de la fixation de l'anticorps IPL Sm1 sur l'antigène 38 KD en phase solide, dont les résultats sont représentés à la Figure 4, est basée sur l'inhibition de la fixation de l'anticorps IPL Sm1 marqué à l'iode 125 à son antigène cible par différents anticorps ou préparations antigéniques.

a) Préparation de la phase solide

Chaque alvéole de plaque de polyvinyle est traitée par 100 µl d'une solution à 10 µg/ml d'anticorps monoclonal C3-109 (anticorps anti-S. mansoni d'isotype IgM qui ne croise pas avec l'épitope de l'anticorps IPL Sm1). Cette méthodologie permet la fixation ultérieure de l'antigène 38 KD. Après 2 heures de contact à 20°C, les plaques sont lavées 3 fois par 200 µl de tampon phosphate 10 mM contenant 0,1% de sérum albumine bovine (BSA), et saturées 30 minutes à 20°C par 200 µl d'une solution à 2% de BSA en tampon phosphate. Les plaques sont ensuite lavées 3 fois en tampon phosphate 10 mM 0,1% BSA et traitées par 100 µl d'un extrait membranaire de schistosomule obtenu selon la technique décrite par DISSOUS et Al. dans Mol. Biochem. Parasitol. (1981), 3, pages 215-225 (100 µl de solution antigénique à 100 µg/ml pour chaque alvéole). Après 2 heures d'incubation à 37°C et 3 lavages par 200 µl de tampon phosphate 10 mM 0,1% BSA, les plaques sont prêtes pour la réaction d'inhibition.

b) Réaction d'inhibition

Cinquante microlitres d'anticorps IPL Sm1 marqué à l'iode 125 (100.000 cpm dans 50 µl), et 50 µl des différents sérums ou fractions antigéniques à tester dilués au 1/50ème en tampon phosphate 10 mM à 0,1% de BSA sont incubés 1 heure à 37°C, puis 1 nuit à 4°C. Les plaques sont alors lavées 3 fois en tampon phosphate 10 mM 0,1% de BSA, et les alvéoles comptées séparément au compteur gamma.

c) Expression des résultats

Le pourcentage d'inhibition de fixation de l'anticorps IPL Sm1 est évalué par rapport à un témoin négatif dans

- 12 -

lequel les 50 µl d'IPL Sm1 marqué sont incubés en présence de 50 µl de tampon phosphate 10 mM 0,1% de BSA.

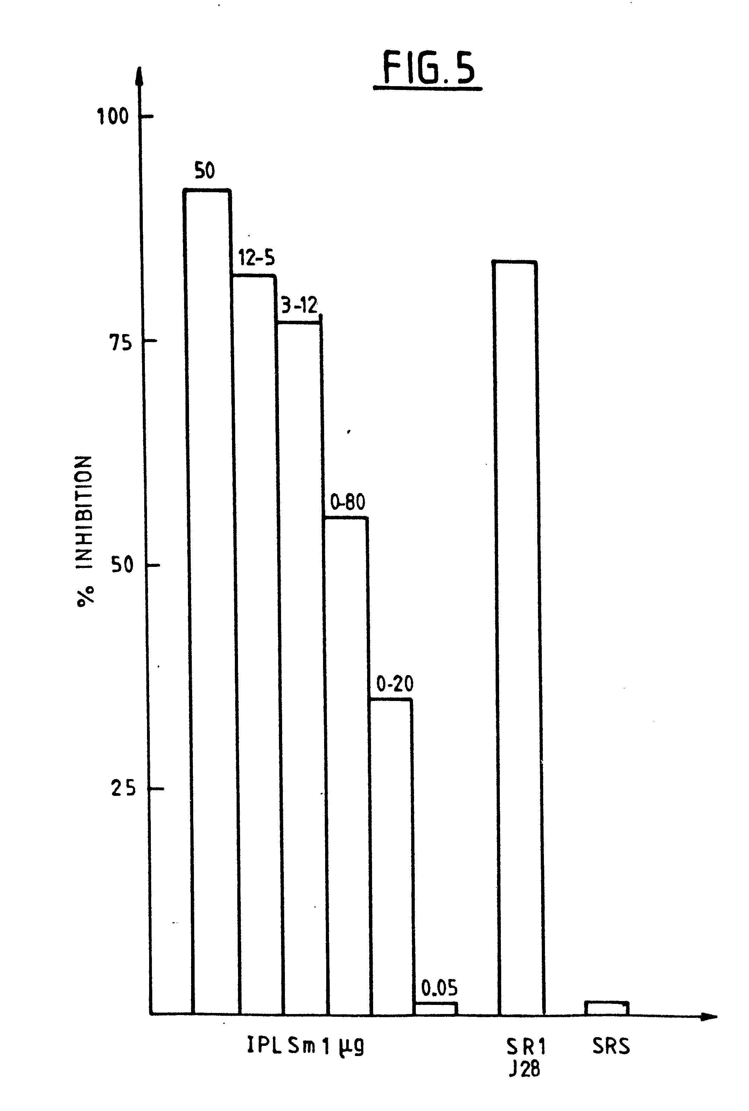

Ces observations ont été d'autre part confortées par la démonstration de l'inhibition de la fixation de l'anticorps IPL Sm1 marqué sur la KLH par différentes doses de l'anticorps IPL Sm1 froid ou par le sérum des rats infectés par S. mansoni représentée à la Figure 5.

La technique radioimmunologique d'inhibition de la fixation de l'anticorps IPL Sm1 sur l'hémocyanine de M. crenulata en phase solide, utilisée dans ce dernier cas, est directement inspirée de la méthode d'évaluation de l'inhibition de la fixation de l'anticorps IPL Sm1 à son antigène cible 38 KD. Dans ce cas particulier, la phase solide est constituée par des plaques de polyvinyle préalablement traitées par de la KLH.

a) Phase solide

Chaque alvéole de plaque de polyvinyle est traitée par 100 µl d'une solution de KLH à 10 µg/ml en tampon phosphate 10 mM à 0,1% de BSA pendant 2 heures à 20°C. Les plaques sont ensuite lavées 3 fois en tampon phosphate 10 mM à 0,1% de BSA et saturées par une solution à 2% de BSA dans le tampon phosphate 10 mM, 30 minutes à 20°C (200 µl par alvéole). L'excès de BSA est éliminé grâce à 3 lavages (200 µl par alvéole) en tampon phosphate 10 mM 0,1% de BSA.

b) Réaction d'inhibition

Elle est réalisée dans les mêmes conditions que celles définies dans le cas du test d'inhibition de la fixation de l'anticorps IPL Sm1 à son antigène 38 KD.

5 - Caractérisation biochimique de l'épitope commun à la KLH et à l'antigène 38 KD

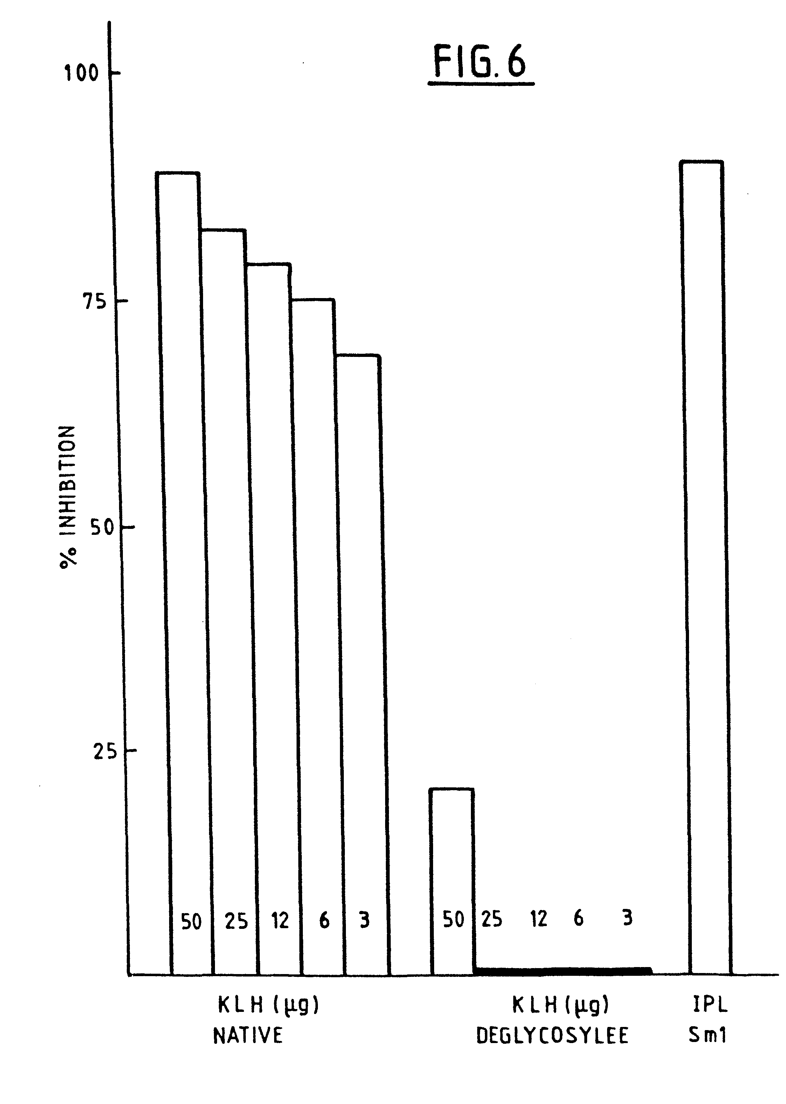

Les premières études effectuées dans le cadre de la caractérisation biochimique de l'épitope spécifique de l'anticorps IPL Sm1 ont démontré grâce aux techniques d'oxydation périodique, la nature glycanique de cet épitope. Des études similaires pratiquées sur la KLH révèlent que le produit de déglycosylation obtenu est incapable d'induire l'inhibition de la fixation de l'anticorps IPL Sm1 à son antigène cible contrairement à la protéine native, ainsi que cela ressort de la Figure 6 annexée qui représente par un graphique un test d'inhibition de la fixation de l'anticorps IPL Sm1 à l'antigène 38 KD par la KLH dont le principe de la technique a été précédemment défini en liaison avec la Figure 4 : 50 µl d'anticorps IPL Sm1 marqué à l'iode 125 (100.000 cpm dans 50 µl) sont incubés respectivement en présence de 50 µl de KLH native, 50 µl de KLH déglycosylée ou 50 µl de l'anticorps IPL Sm1 froid (50 µg).

Les expériences de déglycosylation pratiquées sur la molécule d'hémocyanine de Megathura crenulata par le périodate ou l'acide trifluorométhanesulfonique TMSF (agents de déglycosylation) ont permis d'établir la nature oligosaccharidique de l'épitope reconnu par l'anticorps monoclonal protecteur IPL Sm1, sur la molécule de KLH.

Les expériences d'inhibition de la fixation de l'anticorps IPL Sm1 à son antigène cible 38 KD montrent clairement (cf. Figure 6) que la molécule de KLH déglycosylée ne présente aucune activité inhibitrice de la fixation de l'anticorps IPL Sm1 à son antigène 38 KD, contrairement à la molécule native. L'existence de cette structure sucrée et de son rôle dans l'induction d'une réponse immune anti-S. mansoni a d'autre part été démontrée in vivo lors d'expériences d'immunisation de rats LOU par différentes préparations de KLH native ou déglycosylée. L'étude par immunofluorescence indirecte sur coupe de schistosomule des sérums de ces animaux démontre clairement que seule la KLH native est capable d'induire la production d'anticorps spécifiques de S. mansoni.

Ainsi que cela ressort de ce qui précède, l'invention ne se limite nullement à ceux de ces modes de réalisation et d'application qui viennent d'être décrits de façon plus explicite; elle en embrasse au contraire toutes les variantes qui peuvent venir à l'esprit du technicien en la matière, sans s'écarter du cadre, ni de la portée, de la présente invention.

- 15 -

## REVENDICATIONS

1°) Glycoprotéine extraite de mollusques, savoir l'hémocyanine, qui est caractérisée en ce qu'elle présente les mêmes propriétés antigéniques que l'antigène 38 KD isolé de la surface du schistosomule de S. mansoni.

2°) Oligosaccharide caractérisé en ce qu'il est commun à l'antigène 38 KD du schistosomule et à l'hémocyanine de mollusque, et en ce qu'il est reconnu par un anticorps monoclonal protecteur comme étant l'épitope responsable du pouvoir antigénique de l'antigène 38 KD de schistosomule et de l'hémocyanine de mollusque.

3°) Sérum protecteur à l'égard des infections par Schistosoma mansoni, caractérisé en ce qu'il est constitué par un sérum de mammifère immunisé par de l'hémocyanine de mollusque selon la revendication 1 ou par son épitope oligosaccharidique selon la revendication 2, en ce que ce sérum contient des anticorps capables d'induire en présence d'éosinophiles de mammifères du même genre que ceux dont provient le sérum, des niveaux significatifs de cytotoxicité, de l'ordre de 46 à 94% environ, comparables à ceux obtenus dans le cas des sérums d'infection par S. mansoni ou par l'anticorps monoclonal protecteur précité, lequel sérum est apte à exercer une activité protectrice immunoprophylactique, à l'égard des infections par S. mansoni.

4°) Sérum selon la revendication 3, caractérisé en ce que ce sérum est un sérum de mammifère immunisé par l'hémocyanine de mollusques pris dans le groupe qui comprend notamment Megathura crenulata.

5°) Composition immunogène, caractérisée en ce qu'elle contient en tant que constituant actif une quantité suffisante de la glycoprotéine selon la revendication 1, ou de son épitope oligosaccharidique selon la revendication 2, éventuellement associé(e) à un adjuvant convenable.

6°) Réactif de diagnostic apte à être utilisé pour la séroépidémiologie des bilharzioses humaines, caractérisé

- 16 -

en ce que son constituant essentiel est l'hémocyanine de mollusque, et notamment de mollusques pris dans le groupe comprenant Megathura crenulata, selon la revendication 1 ou son épitope selon la revendication 2.

7°) Vaccin contre les bilharzioses et notamment les bilharzioses humaines, caractérisé en ce qu'il contient une quantité vaccinante active d'hémocyanine de mollusque, et notamment de mollusque pris dans le groupe comprenant Megathura crenulata, selon la revendication 1, ou de l'épitope oligosaccharidique de cette hémocyanine selon la revendication 2.

8°) Agent immunisant, caractérisé en ce qu'il est constitué par un complexe formé par le couplage d'anticorps anti-idiotypes spécifiques d'anticorps monoclonaux de mammifères anti-Schistosoma mansoni, à l'hémocyanine de mollusques, et notamment à l'hémocyanine de Megathura crenulata, selon la revendication 1 ou à son épitope oligosaccharidique selon la revendication 2.

9°) Anticorps monoclonal, caractérisé en ce qu'il est apte à se lier spécifiquement à des structures de nature glycanique de l'hémocyanine de Megathura crenulata, de masse moléculaire 150-200 KD.

10°) Anticorps monoclonal selon la revendication 9, caractérisé en ce qu'il est obtenu à partir d'un hybridome déposé le 25 Avril 1986 sous le N° I-547 auprès de la CNCM et en ce qu'il est désigné par la dénomination "IPL Sm1".

11°) Hybridome utile pour la préparation d'anticorps monoclonaux selon l'une quelconque des revendications 9 et 10, caractérisé en ce qu'il est obtenu par fusion de cellules de rate de rats immunisés contre S. Mansoni et de cellules myélomateuses de rat.

12°) Hybridome selon la revendication 11, caractérisé en ce qu'il a été déposé le 25 Avril 1986 sous le N° I-547 auprès de la CNCM.

13°) Antigène, caractérisé en ce qu'il est reconnu par l'anticorps monoclonal produit par un hybridome selon l'une quelconque des revendications 11 et 12.

14°) Coffret, trousse ou analogue de diagnostic, caractérisé en ce qu'il contient, outre les tampons, supports et autres réactifs usuels permettant la réalisation d'un diagnostic par les méthodes usuelles telles qu'immunofluorescence, EIA, RIA et analogues : - une quantité suffisante de la glycoprotéine immunogène selon la revendication 1 ou de son épitope oligosaccharidique selon la revendication 2; - une quantité suffisante d'un anticorps monoclonal selon l'une quelconque des revendications 9 et 10; - et un sérum de référence.

# FIG. 1

A

B

FIG. 2

0244295

FIG. 3

FIG.4

FIG.5

FIG.6

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X,P | BIOLOGICAL ABSTRACTS/RRM, vol. 32, résumé no. 69731, Philadelphia, US; J.M. GRZYCH et al.: "A protective carbohydrate epitope of schistosoma-mansoni major immunogen is expressed by keyhole limpet hemocyanin KLH", & Joint meeting of the Gesellschaft für Immunologie (Society for Immunology) and the Société Française d'Immunologie (French Society for Immunology), Strasbourg, FR, 19-22 novembre 1986, IMMUNOBIOLOGY 1986. vol. 173, no. 2-5, pages 245-246 | 1,2,9, 10,13 | C 07 K 15/14<br>C 07 H 3/06<br>A 61 K 39/395<br>A 61 K 39/00<br>G 01 N 33/569<br>A 61 K 39/44<br>C 12 P 21/00<br>C 12 N 15/00 |
| | --- | | |
| X,P | NATURE, vol. 323, 2 octobre 1986, pages 443-445, MacMillan Journals Ltd, GB; C. Dissous et al.: "Schistosoma mansoni shares a protective oligosaccharide epitope with freshwater and marine snails" * En entier * | 1-14 | |
| | | | **DOMAINES TECHNIQUES RECHERCHES (Int Cl 4)** |
| | | | A 61 K |
| | ---     -/- | | |

Le présent rapport de recherche a été établi pour toutes les revendications

| Lieu de la recherche | Date d'achèvement de la recherche | Examinateur |
|---|---|---|
| LA HAYE | 14-07-1987 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

| | | Page 2 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X,P | BIOLOGICAL ABSTRACTS, vol. 83, résumé no. 118435, Philadelphia, US; J.M. GRZYCH et al.: "Schistosoma-mansoni shares a protective carbohydrate epitope with keyhole limpet hemocyanin", & JOURNAL OF EXPERIMENTAL MEDICINE (US) 1987. vol. 165, no. 3, pages 865-878 * Abrégé * | 1-14 | |
| | --- | | |
| A | DE-A-2 644 149 (BEHRINGWERKE AG) | | |
| | ----- | | |

DOMAINES TECHNIQUES RECHERCHES (Int. Cl 4)

Le présent rapport de recherche a été établi pour toutes les revendications

| Lieu de la recherche | Date d'achèvement de la recherche | Examinateur |
|---|---|---|
| LA HAYE | 14-07-1987 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82